# EUROPEAN PATENT APPLICATION

(11) **EP 0 629 700 A2**
(43) Date of publication of application: **21.12.1994**
(21) Application number: 94108283.6
(22) Date of filing: 30.05.1994
(51) Int. Cl.: C12N 15/85, C12N 5/16, C12N 15/69, C12N 15/90

(54) **Vector and mammalian cell line having improved productivity**

(30) Priority: 10.06.1993 US 75209
(71) Applicant: Bayer Corporation, Pittsburgh, PA 15219-2502 (US)
(72) Inventor: Chan, Sham-Yuen, El Sobrante, California 94803 (US)
(74) Representative: Dänner, Klaus, Dr.

(57) **Abstract**

An improved vector for introducing a DNA coding sequence for a heterologous protein into a mammalian cell line to increase protein expression. The vector comprises a cDNA protein coding sequence, a first promoter and a downstream donor intron, the first promoter and downstream donor intron being located between a second promoter and a donor intron acceptor. In an illustrative example, the sequence codes for Factor VIII and high productivity of a transfected cell line is assured by establishing a minimum amplifiability index which, contrary to current practice, focuses on using cell lines having relatively low productivity prior to amplification.

SEQUENCE LISTING

(1) GENERAL INFORMATION:

(i) APPLICANT:
(A) NAME: Miles Inc
(B) STREET: 500 Grant Street
(C) CITY: Pittsburgh
(D) STATE: PA
(E) COUNTRY: USA
(F) POSTAL CODE (ZIP): 15219-2502
(G) TELEPHONE: 0214/3061450
(H) TELEFAX: 0214/303482

(ii) TITLE OF INVENTION: Vectors and Mammalian Cell Line having
improved Productivity

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: US 08/075,209

(2) INFORMATION FOR SEQ ID NO: 1:

(i) SEQUENCE CHARACTERISTICS:
(A) LENGTH: 382 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 1:

CCGCGGTCCT CCTCGTATAG AAACTCGGAC CACTCTGAGA CGAAGGCTCG CGTCCAGGCC 60

AGCACGAAGG AGGCTAAGTG GGAGGGGTAG CGGTCGTTGT CCACTAGGGG GTCCACTCGC 120

TCCAGGGTGT GAAGACACAT GTCGCCCTCT TCGGCATCAA GGAAGGTGAT TGGTTTATAG 180

GTGTAGGCCA CGTGACCGGG TGTTCCTGAA GGGGGGCTAT AAAAGGGGGT GGGGGCGCGT 240

TCGTCCTCAC TCTCTTCCGC ATCGCTGTCT GCGAGGGCCA GCTGTTGGGG TGAGTACTCC 300

CTCTCAAAAG CGGGCATGAC TTCTGCGCTA AGATTGTCAG TTTCCAAAAA CGAGGAGGAT 360

TTGATATTCA CCTGGCCCGC GG 382

## Description

### BACKGROUND OF THE INVENTION

Field: This invention relates generally to the production of biologically-active proteins from genetically-engineered mammalian cell lines. Specifically, the invention is concerned with a novel vector and cell selection method which enhances cell line productivity. The invention is especially useful for increased production of recombinant Factor VIII.

Prior Art: Gene amplification is a strategy which has been broadly applied to increase protein production in mammalian cells. A transcription unit (cDNA) encoding a protein of interest is normally linked covalently to an amplifiable marker. The transcription unit and marker are then co-transferred into an appropriate cell, followed by selection and co-amplification.

The most widely used gene transfection and amplification strategy involves the use of dihydrofolate reductase (DHFR) expression vectors in conjunction with DHFR-deficient Chinese hamster ovary (CHO) cells (Kellems, Curr. Op. Biotech. 2:723-729, 1991). A number of commercially valuable proteins including tissue plasminogen activator, erythropoietin, and factor VIII have been produced by this strategy (see U.S. 4,740,461 to Kaufman and Kaufman, et al., Mol. Cell. Biol. 5(7): 1750-1759, 1985). Due to the presence of the endogenous DHFR gene, this strategy has generally not been extended to cell hosts that are not DHFR-deficient. However, limited success has been reported where DHFR⁺ cell hosts were used to express therapeutic proteins.

U.S. patent 4,965,199 (Wood, et al., 1990) describes a process for producing factor VIII in DHFR⁺ baby hamster kidney cells (BHK-21). This process involves (i) the co-transfection of host cells with a DNA sequence coding for factor VIII linked to a 2nd DNA sequence encoding DHFR, and a selectable marker conferring neomycin resistance, (ii) selection of transfectants in a selective medium containing G418, and (iii) amplification of G418 resistant cells in media containing increasing amounts of methotrexate (MTX).

Wall, et al. (Gene, 81: 139-149, 1989) have reported on the use of the DHFR/MTX co-amplification strategy to express functional protein C in human 293 cells. Okamoto, et al. (Biotechnol., 8: 550-553, 1990) have also described the amplification and expression of granulocyte colony stimulating factor (GM-CSF) in human lymphoid cells.

Despite recent advances in recombinant DNA techniques, the derivation of recombinant cell clones with high productivity for factor VIII has remained difficult. European patent application No. 0260148 (Gorman, published September 17, 1987) and incorporated herein by reference describes the construction of an expression vector (pCIS-F8) having a stabilizing sequence downstream of a promoter and upstream of the DNA encoding factor VIII. The vector was constructed with a cytomegalovirus or CMV promoter and enhancer, a cDNA encoding Factor VIII and a 3' terminating sequence. In the absence of this stabilizing sequence, expression of factor VIII was not observed in any cell types tested.

By co-transfecting human 293S cells with pCIS-F8 and a plasmid expression vector which confers neomycin resistance, recombinant cell lines with moderate productivity (0.1 - 0.2 uU/c/d) for factor VIII were obtained. However, surprisingly numerous attempts to amplify a large number of populations and clones from various transfection experiments failed to generate high producing clones after extensive amplification in methotrexate (up to 1 - 2 uM). The amplification was done as described by U.S. Patents 4,740,461(Kaufman) and 4,965,199 (Wood, et al.).

It has now been found that the expression level of a protein such as Factor VIII can be significantly increased by modifying a vector used to transfect a mammalian cell. Details of the modification and its use are described below.

### SUMMARY OF INVENTION

The vector of this disclosure comprises a protein coding sequence, a first promoter and a downstream donor intron, the first promoter and downstream donor intron being located between a second promoter and a donor intron acceptor. In one preferred embodiment the sequence codes for Factor VIII (also known as FVIIIC or FVIII:C) and the vector is pCAIS-F8. The coding sequence can also be for Factor VIII derivatives, variants or fragments (see, for example, U.S. Patent 5,045,455). The vector is used to introduce the appropriate DNA coding sequence for an heterologous protein into mammalian cells to increase protein expression by the cells.

This disclosure also describes an improved process to select for amplifiable cell populations for desired protein such that continuous production of the protein can be achieved and with higher productivity.

A preferred amplifiable marker is DHFR although other markers such as glutamine synthetase (GS) and multidrug-resistance (MDR) can be substituted. The cell host to be transfected can be any mammalian cell and need not be DHFR-deficient. Cell lines that are known to integrate selection genes into their chromosome DNA are best; for example, human embryonic kidney (293S), Chinese hamster ovary (CHO), baby hamster kidney (BHK-21), myeloma, and hepatoma lines such as HepG2 cells, and the like.

The productivity of a transfected mammalian cell line can be enhanced by establishing a minimum amplifiability index which, contrary to current practice, focuses on using cell lines having relatively low productivity after an initial selection and mini-cloning, but prior to amplification. According to the disclosure, selected cells must have an amplifiability index $\text{(ΔE = Y/X) >3}$ where X = production of the cells before the first amplification and Y = production of the cells after the first amplification.

### BRIEF DESCRIPTION OF THE FIGURES

Figure 1a shows the prior art vector pCIS-F8.

Figure 1b shows the vector pCAIS-F8 of this disclosure.

Figure 2a shows a linear map of the CMV promoter donor₂-intron₂-acceptor (D2I2A) of Figure 1a.

Figure 2b shows a linear map of the location of AML promoter and donor¹-intron¹ in relation to CMV promoter and donor²-intron²-acceptor of Figure 1b.

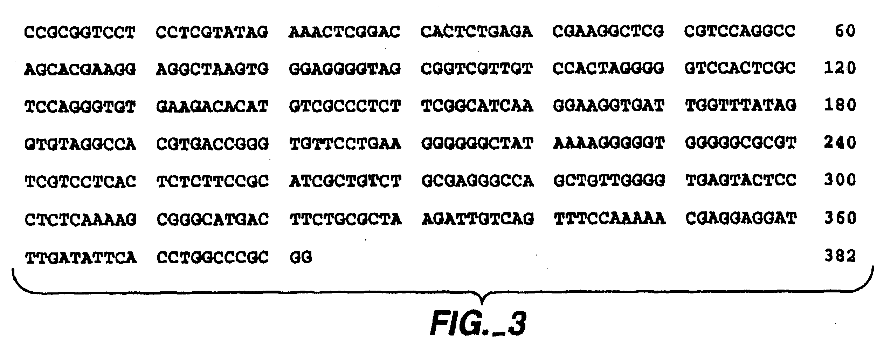

Figure 3 shows the nucleotide sequence of adeno major late promoter and its intron.

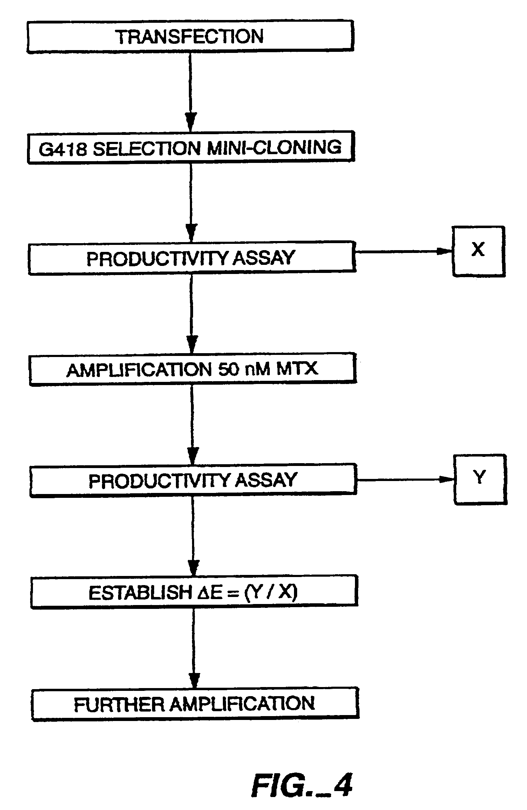

Figure 4 is a flow chart illustrating the overall selection process of this disclosure and the calculation of ΔE.

Figure 5 is a flow chart illustrating the construction scheme of the vector pCAIS-F8.

### SPECIFIC EMBODIMENTS

### Example 1. Construction of expression vectors.

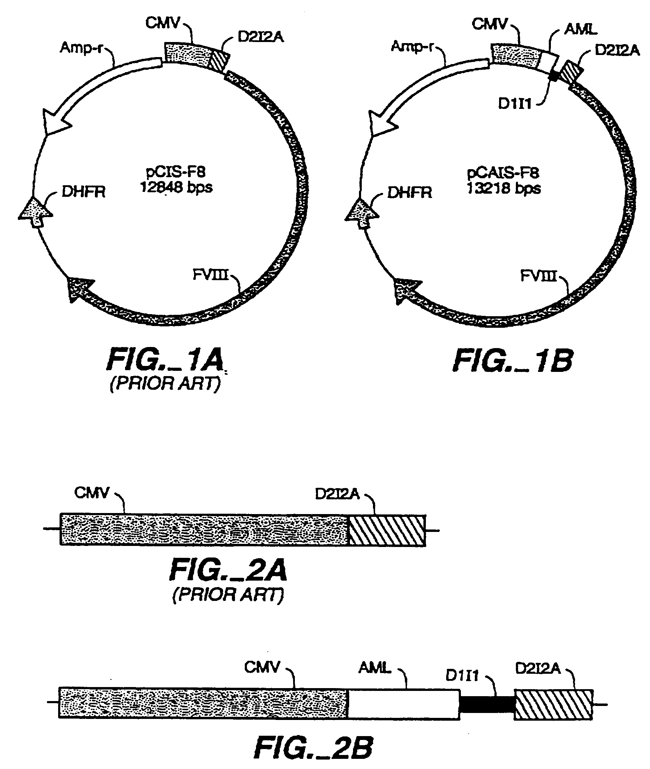

Expression vectors pCIS-F8 and pSV₂-neoBa16 were constructed as described in European Patent Application No. 0260148. The improved expression vector (designated pCAIS-F8) was constructed by inserting the adeno major late promoter including the immediate 3' intron sequence (+1 to +132 with respect to the CAP site, Fig. 2) into the SacII site of the known vector pCIS-F8 using standard recombinant technique (Maniatis, 1990). This 382 bp SacII fragment was isolated from pAML3p.8cl as described in U.S. Patent 4,965,199 to Wood, et al.

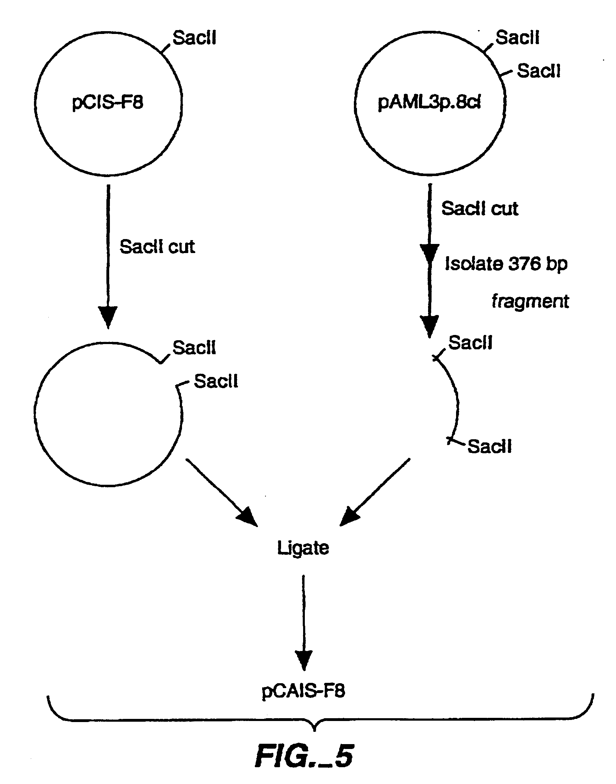

Briefly, a 382 bp fragment was gel-purified after digesting 50ug of pAML3p.8cl with SacII. This fragment was then ligated to pCIS-F8 which has been linearized with SacII. After 4 hours of ligation at 20 degree C, 50 ng of the ligation mix was used to transform E. coli DH5α. Successful cloning of the AML promoter and intronic sequences was confirmed by sequencing (Fig. 2). The construction scheme of the vector is shown in Figure 5.

### Example 2. Derivation of stable cell clones with high productivity.

Production cell lines were established by co-transfecting 5 x 10⁵ cells with 10 ug of pCIS-F8 or pCAIS-F8 and 10 ug of pSV₂-neoBa16 using the lipofection method according to manufacturer's instructions. A typical transfection experiment involves 2.5 x 10⁶ cells (GIBCO). Multiple transfections are normally done simultaneously. Two days following transfection, cells were trypsinized, seeded into a 48-well plate (CoStar, growth area = 100 mm²), and grown in a selective medium (DMEM/F12, weight ratio = 1:1) containing G418 (400 ug/ml). The factor VIII productivity of the G418-resistant cells were assayed at ∼50% confluency (about 3 weeks after the onset of selection) by a chromogenic substrate method according to manufacturer's instructions (Coatest, Kabi). The cells were then allowed to grow to confluency in a medium (DMEM/F12, 1:1, deficient in glycine, hypoxanthine, and thymidine) containing 50 nM methotrexate. This initial amplification step normally takes about 3 weeks. The methotrexate-resistant cells were then assayed for factor VIII productivity.

### CELL LINE SELECTION PROCESS

A "delta E index"(ΔE) of each population was then determined. A ΔE index is defined as $\text{ΔE = Y/X}$ where X = productivity before 50 nM methotrexate selection and Y = productivity after 50 nM methotrexate selection. A typical transfection would involve the screening of 220 - 440 populations of transfected cells. Cell populations with ΔE >3 were further subcloned and amplified in the next higher level of methotrexate (100 nM).

It is to be noted that populations with high productivity (which according to prior art would have been picked for further amplification) fail to be amplified to higher productivity. All these populations have a low ΔE index value of less than 3. Cell populations with moderate productivity but a high ΔE index (>3) were amplified to higher productivity.

A typical selection involves the subculturing of 1 population (∼1 x 10⁵) of cells into 96 subpopulations (96-well plate, growth area = 32 mm²). The cells were grown to confluency in 100 nM methotrexate and assayed for factor VIII productivity. The population with the highest productivity was then subjected to the same regimen of clonal enrichment under amplification in the next higher level of methotrexate (200 nM). The same regimen of selection was repeated until a steady high level of factor VIII productivity was reached. For 293S cells maximum productivity is typically achieved at 1 uM methotrexate. Further amplification in higher level of methotrexate did not increase factor VIII productivity. Single cell clones were then obtained by limiting dilution cloning in the presence of 50 nM methotrexate.

As shown in Table 1, clones with high productivity were obtained from both pCIS-F8 (0.7 - 1.0 uU/c/d) and pCAIS-F8 1.3 - 1.5 uU/c/d). Clones generated by pCAIS-F8 showed a significant 40 -50% in productivity over those generated by pCIS-F8. These clones are stable and suitable for continuous production of factor VIII under serum-free conditions.

**TABLE I**

| Factor VIII productivity of amplified clones of 293S cells transfected with various expression vectors. | | |
|---|---|---|
| Vectors (1uM) | Screening Method | Productivity of Clones** (uU/c/d) |
| pCIS-F8 | Traditional* | 0.1 - 0.25 |
| pCIS-F8 | ΔE | 0.7 - 1.0 |
| pCAIS-F8 | Traditional* | 0.15 - 0.3 |
| pCAIS-F8 | ΔE | 1.3 - 1.5 |

| | | |
|---|---|---|
| *Cells showing highest productivity after G418 selection were chosen for further amplification in methotrexate. | | |
| **A total of 10-12 clones was evaluated by productivity after attaining resistance to 1uM methotrexate. | | |

As shown in this disclosure, the Δ E index is a useful indicator to identify potential high producing cell populations that can be further amplified.

### Example 3. Continuous production of factor VIII.

One of the high producing clones were grown to a cell density of 5 - 7 x 10⁵ cells/ml in a 250 ml spinner in Joklik's minimum essential medium (GIBCO) supplemented with 10% dialyzed fetal bovine serum. The culture was stirred at a speed of 100 r.p.m. and kept in a 37° C incubator. The cells were then washed with Hank's buffered saline (GIBCO), re-seeded in a 250 ml spinner flask at a density of 5 x 10⁵ cells/ml in a serumfree production medium (Joklik's MEM supplemented with insulin, 5 ug/ml, transferrin 25 ug/ml, human serum albumin, 1 mg/ml, and 15 mM MgCl₂), and kept at 37°C. After 24 hours, the culture fluid was harvested and assayed for factor VIII production by the chromogenic Coatest (Kabi) method. The cells were spun down, re-suspended in fresh serumfree production medium, and incubated at 37°C for another 24 hours after which the same procedure is repeated at 24 hours intervals for 21 days. Throughout this production period the productivity of the cells was maintained in the range of 1.2 - 1.5 uU/c/d.

Given the above disclosure, variations will occur to those skilled in this field. For example, the arrangement of the augmenting sequence and the vectors of this disclosure could be used as vectors intended for gene therapy in humans. In the case of Factor VIII, the vector could be integrated into human liver cells. Accordingly, it is intended that the above examples should be construed as illustrative and that the scope of the invention disclosed should be limited only by the following claims.

## Claims

1. A vector comprising a protein coding sequence, a first promoter and a downstream donor intron, the first promoter and downstream donor intron being located between a second promoter and a donor intron acceptor.

2. The vector of claim 1 comprising a coding sequence for Factor VIII or a Factor VIII derivative located downstream from the donor intron acceptor.

3. A mammalian cell line comprising the vector of claim 1 as an integrated chromosomal element.

4. A mammalian cell line comprising the vector of claim 2 as an integrated chromosomal element.

5. The vector pCAIS-F8 chromosomally integrated into a mammalian cell.

6. A process of selecting a cell line for producing a heterologous protein, comprising:
A. cotransfecting mammalian cells with a DNA sequence coding for the heterologous protein, a second DNA sequence encoding an amplifiable marker, and a third DNA sequence encoding a selectable marker;
B. further cloning the transfected cells into subpopulations in a selected medium and selecting for such selectable marker resistant cells;
C. assaying for heterologous protein productivity;
D. culturing said selected marker resistant cells in media containing a low level of that selective agent, wherein the host cell is not deficient in the amplifiable marker;
E. establishing the amplifiability index ΔE for each cell population, said index being defined by the equation:
$\text{ΔE = Y / X}$
wherein X is the production of cells before a first amplification, and Y is the production of cells after said first amplification; and
F. further amplifying cells with a ΔE index value of greater than 3, said amplifying step being performed in increasing amounts of selective agent.

7. The method of claim 6 wherein said host cells comprise an expression vector having a sequence of double stranded DNA including:
A. an augmenting sequence downstream of a promoter and upstream of a DNA encoding the amino acid sequence of the desired heterologous protein;
B. DNA encoding the amino acid sequence of the desired protein downstream of said augmenting sequence; and
C. DNA coding a polyadenylation sequence downstream of a transcription termination site.

8. The method of claim 7 wherein said host cells further comprise an augmenting sequence.

9. The method of claim 7 wherein the augmenting sequence comprises a splice donor₁ - intron₁ - splice donor₂ - intron₂ - acceptor sequence.

10. The method of claim 7 wherein the promoter is from the immediate early gene of human cytomegalovirus.

11. The method of claim 7 wherein the splice₁ - intron₁ sequence is from the major late promoter region of adenovirus.

12. The method of claim 7 wherein the splice donor₂ sequence is from the human cytomegalovirus.

13. The method of claim 7 wherein the intron₂ - acceptor sequence corresponds to the human immunoglobulin variable region.

14. The method of claim 7 wherein the DNA coding the amino acid sequence of a heterologous protein encodes factor VIII.

15. The method of claim 7 wherein the DNA coding the amino acid sequence of a heterologous protein encodes derivatives, variants or fragments of factor VIII.

16. The method of claim 7 wherein the host cell is human embryonic kidney cell (293).
